# EUROPÄISCHE PATENTSCHRIFT

(11) **EP 1 140 349 B1**
(45) Veröffentlichungstag und Bekanntmachung des Hinweises auf die Patenterteilung: **07.08.2002**
(21) Anmeldenummer: 99959315.5
(22) Anmeldetag: 23.11.1999
(51) Int. Cl.: B01J 8/22, B01J 10/00, B01J 19/24

(54) **REAKTOR ZUR KONTINUIERLICHEN DURCHFÜHRUNG VON GAS-FLÜSSIG-, FLÜSSIG-FLÜSSIG- ODER GAS-FLÜSSIG-FEST-REAKTIONEN**
REACTOR FOR CARRYING OUT GAS-LIQUID, LIQUID-LIQUID OR GAS-LIQUID-SOLID CHEMICAL REACTIONS
REACTEUR POUR EFFECTUER EN CONTINU DES REACTIONS GAZ-LIQUIDE, LIQUIDE-LIQUIDE OU GAZ-LIQUIDE-SOLIDE

(30) Priorität: 26.11.1998 DE 19854637
(43) Veröffentlichungstag der Anmeldung: 10.10.2001
(73) Patentinhaber: BASF AKTIENGESELLSCHAFT, 67056 Ludwigshafen (DE)
(72) Erfinder: BERG, Stefan, D-67227 Frankenthal (DE); ZEHNER, Peter, D-67074 Ludwigshafen (DE); BENFER, Regina, D-67122 Altrip (DE); MÜLLER, Jörn, D-67071 Ludwigshafen (DE); NILLES, Michael, D-67061 Ludwigshafen (DE); SCHULZ, Ralf, D-67346 Speyer (DE); STÜTZER, Dieter, D-67373 Dudenhofen (DE)
(74) Vertreter: Isenbruck, Günter, Dr.
(86) Internationale Anmeldenummer: EP9909059
(87) Internationale Veröffentlichungsnummer: WO0030743

(56) Entgegenhaltungen:
- GB-A- 1 013 888
- US-A- 3 723 545
- US-A- 4 234 560
- US-A- 5 154 898
- PATENT ABSTRACTS OF JAPAN vol. 012, no. 258 (C-513), 20. Juli 1988 (1988-07-20) & JP 63 043920 A (HITACHI LTD;OTHERS: 01), 25. Februar 1988 (1988-02-25)

## Beschreibung

Die Erfindung betrifft einen Reaktor zur kontinuierlichen Durchführung von Gas-Flüssig-, Flüssig-Flüssig- oder Gas-Flüssig-Fest-Reaktionen, ein Verfahren sowie eine Verwendung.

In vielen chemischen Verfahren sind der Gas-Flüssigkeits-Stoffübergang und die Wärmeabfuhrleistung die geschwindigkeitsbestimmenden Schritte. Zur Verbesserung des Stoffübergangs an der Phasengrenzfläche wurde für derartige Reaktionen die Verwendung von Ejektoren vorgeschlagen, d.h. von Vorrichtungen, die die kinetische Energie eines Flüssigkeitsstrahls hoher Geschwindigkeit zum Ansaugen und Dispergieren der Gasphase nutzen. Durch den hohen Energieeintrag wird im Ejektor eine hohe Turbulenz und eine große Scherkraft erzeugt mit der Folge, daß das Gas in Form sehr kleiner Bläschen dispergiert wird, d.h. daß eine sehr hohe volumenspezifische Gas-Flüssigkeits-Grenzfläche erzeugt wird. In der Literatur werden für die volumenspezifische Phasengrenzflächen Werte von 40.000 bis 70.000 m²/m³ im Ejektor beschrieben gegenüber 500 bis 2.500 im gesamten System, d.h. außerhalb des Ejektors (vgl. Chem. Eng. Sci. Vol. 47, No. 13/14 pp. 3557 ff, 1992).

Vorrichtungen zur Durchführung von Gas-Flüssig- oder Gas-Flüssig-Fest-Reaktionen unter Verwendung von Ejektoren sind als Loop-Venturi-Reaktoren bekannt und beispielsweise in

DE-A-4323687 in Verbindung mit einem kontinuierlichen Verfahren zur Herstellung von aromatischen Di- und Polyaminen durch katalytische Hydrierung der entsprechenden Polynitroverbindungen beschrieben. An einem Ende des Loop-Venturi-Reaktors befindet sich der Ejektor, d.h. eine Zweistoff-Strahldüse mit fest angeordnetem Impulsaustauschrohr und Diffusor, worin flüssiges Umpumpgemisch und gasförmige Reaktanden (Wasserstoff) in Kontakt gebracht werden. Der Ejektor taucht zu einem gewissen Anteil seiner Baulänge durch den Gas-Flüssig-Phasentrennspiegel in die Flüssigkeit ein. Im Ejektor selbst ist die Gasdispergierleistung hoch, nicht jedoch im übrigen Reaktorvolumen, das bezüglich des Strömungs- und Verweilzeitverhaltens reine Blasensäulencharakteristik aufweist. In diesem Reaktorvolumen außerhalb des Ejektors halten regellose klein- und großräumige Wirbel mit vergleichsweiser geringer Stoffübergangsleistung den Katalysator in suspendiertem Zustand. Der Gasgehalt im Reaktor stellt sich überwiegend aufgrund der Stoffeigenschaften, in geringem Maße auch aufgrund der Anfangsdispergierung ein. Die freiwerdende Reaktionswärme wird über einen externen Wärmetauscher im Umpumpkreislauf abgeführt.

In EP-A-263 935 werden Rührkesselreaktoren für die Durchführung stark exothermer Reaktionen beschrieben, wobei die freiwerdende Wärme über Fieldrohr-Wärmetauscher am Ort Ihrer Entstehung abgeführt wird. Fieldrohr-Wärmetauscher bezeichnen in bekannter Weise Wärmetauscher, die ein Bündel paralleler Doppelmantelrohre aufweisen, wobei die in den Reaktorraum ragenden Enden der Außenrohre geschlossen und die entsprechenden Enden der Innenrohre offen sind, dergestalt, daß der Wärmeträger über einen außerhalb des Reaktorraums angeordneten Zuführraum in die Innenrohre einströmt und über den Raum zwischen Innen- und Außenrohren sowie einen Abführraum ausströmt. Sie sind durch ein hohes Verhältnis von Wärmetauscherfläche zu Volumen des Reaktionsraumes gekennzeichnet und sind somit besonders effektiv für die Abfuhr der freiwerdenden Reaktionswärme. Das beschriebene Verfahren hat jedoch den Nachteil, daß die Phasendurchmischung nicht sichergestellt ist, mit der Folge, daß verstärkt unkontrollierbare Nebenreaktionen unter Ausbeuteverminderung ablaufen und daß sich die Kühlflächen mit harzartigen Verbindungen und/oder Katalysatoranteilen belegen.

Aufgabe der Erfindung ist es daher, einen Reaktor für Gas-Flüssig-, Flüssig-Flüssig- oder Gas-Flüssig-Fest-Reaktionen zur Verfügung zu stellen, der eine intensive Phasendurchmischung im gesamten Reaktionsvolumen und somit eine erhöhte Wirtschaftlichkeit und eine verbesserte Raum-Zeit-Ausbeute sicherstellt.

In einer Ausgestaltung ist es Aufgabe der Erfindung, eine weitgehende Isothermie des Reaktors zu gewährleisten, d.h. einen sehr kleinen Temperaturgradienten über die Reaktorhöhe.

Weiterhin ist es Aufgabe der Erfindung, ein kontinuierliches Verfahren für Gas-Flüssig-, Flüssig-Flüssig- oder Gas-Flüssig-Fest-Reaktionen unter Verwendung eines erfindungsgemäßen Reaktors bereitzustellen.

Die Erfindung geht aus von einem Reaktor für Gas-Flüssig-, Flüssig-Flüssig- oder Gas-Flüssig-Fest-Reaktionen in hochzylindrischer Bauform mit einer im oberen Reaktorbereich angeordneten, nach unten gerichteten Strahldüse, über die die Edukte und das Reaktionsgemisch zugeführt werden sowie mit einem Abzug bevorzugt im unteren Reaktorbereich, über den das Reaktionsgemisch in einem äußeren Kreislauf mittels einer Pumpe der Strahldüse erneut zugeführt wird.

Die Lösung ist dann dadurch gekennzeichnet, daß im Reaktor ein konzentrisches Leitrohr angeordnet ist, das sich im wesentlichen über die gesamte Länge des Reaktors mit Ausnahme der Reaktorenden erstreckt und eine Querschnittsfläche im Bereich von einem Zehntel bis zur Hälfte der Querschnittsfläche des Reaktors aufweist, und daß die Strahldüse oberhalb des oberen Endes des Leitrohrs, bevorzugt um ein Achtel des Leitrohrdurchmessers bis zu einem Leitrohrdurchmesser beabstandet, angeordnet ist oder in das Leitrohr, in eine Tiefe bis zu mehreren Leitrohrdurchmessern, eintaucht, und daß in den Ringraum ein Wärmetauscher mit Wärmetauschrohren, integriert ist, wobei die Wärmetauschrohre parallel zum Leitrohr angeordnet sind.

Es wurde demgemäß gefunden, daß die Stoff- und Wärmetransportbegrenzung weitgehend bis vollständig ausgeschaltet wird, und daß somit die Gesamtreaktion rein kinetisch kontrolliert abläuft.

Die erfindungsgemäße Lösung, wonach der überwiegende Teil des Reaktionsgemisches in einer gerichteten internen Schlaufenströmung geführt und lediglich ein geringer Anteil des Reaktionsgemisches, der für den Antrieb der Schlaufenströmung notwendig ist, extern umgepumpt wird, ist energetisch vorteilhaft.

Dadurch, daß im gesamten Reaktorvolumen die Strömungsbedingungen eindeutig definiert sind, d.h. daß die für die Auslegung des Reaktors wichtigen Größen: Strömungsgeschwindigkeiten, Gasgehalte, Rückvermischung, Mischzeit und Verweilzeitverhalten ausreichend präzise erfaßt werden können, ist der erfindungsgemäße Reaktor unmittelbar scale-up-fähig.

Der Begriff Strahldüse bezeichnet in bekannter Weise ein sich in Strömungsrichtung verjüngendes Rohr; die Strahldüse kann als Dreistoff- oder Zweistoffdüse, gegebenenfalls mit Impulsaustauschrohr und Diffusor oder als Einstoffdüse ausgebildet sein.

Bei der Verwendung einer Einstoffdüse kann zusätzlich die Einleitung eines oder mehrerer gasförmiger Reaktanden in den Gasraum am Reaktorkopf oder über geeignete Vorrichtungen, bevorzugt über ein oder mehrere, insbesondere ein bis drei Ringrohre, mit einer Vielzahl von Öffnungen, insbesondere im unteren Reaktorbereich oder über die Reaktorhöhe verteilt, in den Ringraum zwischen Leitrohr und Reaktorinnenwand erfolgen. Die Gaszufuhr kann jedoch auch unterhalb der Prallplatte oder direkt in den Gasraum oberhalb der Einstoffdüse erfolgen. Der durch die Gasblasen erzeugte Auftrieb unterstützt den Antrieb der internen Schlaufenströmung.

Der Abzug für das Reaktionsgemisch kann grundsätzlich auf jeder beliebigen Reaktorhöhe, bevorzugt im unteren Reaktorbereich, besonders bevorzugt am Boden des Reaktors angeordnet sein. Über den Abzug wird ein Teil des Reaktionsgemisches mittels einer Pumpe abgezogen und gegebenenfalls nach Abtrennung fester Anteile, insbesondere von suspendiertem Katalysator, beispielsweise in einem Querstromfilter, der Strahldüse im oberen Reaktorteil erneut zugeführt.

Der Begriff Leitrohr bezeichnet vorliegend ein im vertikalen zylindrischen Reaktor konzentrisch angeordnetes Innenrohr, das sich nahezu über die gesamte Reaktorlänge mit Ausnahme der Reaktorenden erstreckt. Das obere Leitrohrende muß stets mit Flüssigkeit bedeckt sein, d.h. es muß sich unterhalb des oberen Gasabscheideraums befinden, wobei dieser erfindunggemäß etwa 3 bis 15% des Gesamtvolumens des Reaktors, insbesondere 5 bis 7% des Gesamtvolumens des Reaktors einnimmt. Das obere Ende des Leitrohrs muß von der nach unten gerichteten und bevorzugt auf derselben Vertikalen wie das Leitrohr angeordneten Strahldüse beabstandet sein. Durch die relative Positionierung von Düse/Leitrohr und dem inneren Füllstand kann der Gasgehalt im Reaktionsgemisch eingestellt werden.

Die Strahldüse mündet bevorzugt im Bereich des Trennspiegels Gas-Flüssigkeit. In diesem Fall kann der Gasgehalt im Reaktionsvolumen durch Anheben oder Absenken des Innenstands in gewissen Grenzen verändert werden. Wird beispielsweise die Düse durch Anheben des Innenstands in die Flüssigkeit eingetaucht, so nimmt der Gasgehalt im Reaktionsgemisch kleinere Werte an, wogegen durch Absenken des Innenstands unterhalb der Mündung der Strahldüse größere Gasmengen in das Reaktionsvolumen angesaugt werden.

Das Leitrohr weist eine Querschnittsfläche bevorzugt im Bereich von einem Zehntel bis zur Hälfte der Querschnittsfläche des Reaktors auf. Das untere Ende des Leitrohrs endet im unteren Ende des Reaktorbereichs, bevorzugt im Bereich des zylindrischen Endes des Reaktors.

Im Ringraum zwischen Leitrohr und Reaktorinnenwand ist ein Wärmetauscher, insbesondere ein Wärmetauscher mit zwischen Böden eingeschweißten Wärmetauscherrohren, die vorzugsweise parallel zum Leitrohr angeordnet sind, integriert. Dadurch wird besonders für Reaktionen mit großer Wärmetönung eine hohe Isothermie des Reaktors gewährleistet, d.h. ein sehr kleiner Temperaturgradient über die Reaktorhöhe, da die Reaktionswärme am Ort ihrer Entstehung abgeführt wird. Zudem ist die Sicherheit des Verfahrens gegenüber einer Kühlung im äußeren Kreislauf verbessert, da die Reaktorkühlung auch dann noch funktioniert, wenn die Pumpe für den äußeren Kreislauf ausfällt. Bei Ausfall der Kühlwasserversorgung funktioniert die Kühlung noch bis zur Verdampfung des gesamten Kühlwassers in den Wärmetauscherrohren.

Es ist jedoch auch möglich, in den Ringraum Wärmetauscher von anderer Bauform, insbesondere Fieldrohr-Wärmetauscher, zu integrieren.

Der Reaktor weist in bevorzugter Weise einen Schlankheitsgrad, d.h. ein Verhältnis von Länge (1) zu Durchmesser (d), von 3 bis 10 bevorzugt von 6 bis 10, auf. Der Reaktor kann mit diesem hohen Schlankheitsgrad aufgrund der gerichteten internen Schlaufenströmung gebaut werden. Als Folge des hohen Schlankheitsgrades kann der Gasgehalt im Reaktionsvolumen bereits durch kleine Änderungen des Flüssigkeitsinnenstands im Reaktor verändert werden, weil dadurch die relative Positionierung von Strahldüse und Gas-Flüssigkeits-Trennspiegel verändert wird.

Das Verhältnis der Durchmesser von Leitrohr und Reaktor liegt bevorzugt im Bereich von 0,25 bis 0,5, besonders bevorzugt im Bereich von 0,28 bis 0,33.

In bevorzugter Weise ist im unteren Reaktorbereich, unterhalb des unteren Endes des Leitrohrs, von diesem bevorzugt um einen bis zwei Leitrohrdurchmesser beabstandet, im wesentlichen senkrecht zum Leitrohr, eine Prallplatte angeordnet. Die Prallplatte ist bevorzugt scheibenförmig ausgebildet, mit einem Durchmesser größer als der Durchmesser des Leitrohrs und kleiner als der Innendurchmesser des Reaktors und mit einer Dicke, die durch die mechanische Festigkeit des bevorzugt metallischen Werkstoffs bestimmt ist, d.h. im Bereich von etwa 5-10 mm. Die Prallplatte hat neben der Funktion der Strömungsumkehr die Aufgabe, zu verhindern, daß Gasblasen in den äußeren Kreislauf mitgerissen werden und die Pumpe beschädigen.

Erfindungsgemäß wird auch ein kontinuierliches Verfahren zur Durchführung von Gas-Flüssig-, Flüssig-Flüssig oder Gas-Flüssig-Fest-Reaktionen wie in einem vorstehend beschriebenen Reaktor zur Verfügung gestellt, wobei der überwiegende Teil des Reaktionsgemisches, entsprechend dem 2- bis 30-fachen, bevorzugt dem 5- bis 10-fachen des Volumenstroms des extern umgepumpten Reaktionsgemisches in einer internen Schlaufenströmung das Leitrohr von oben nach unten und den Ringraum zwischen Leitrohr und Reaktorinnenwand von unten nach oben durchströmt. Die interne Schlaufenströmung bezeichnet vorliegend einen Kreislauf des Reaktionsgemisches innerhalb des Reaktors; sie wird durch den Flüssigkeitsstrahl angetrieben, den die im oberen Reaktorbereich angeordnete, nach unten gerichtete Strahldüse erzeugt. Dieser nach unten gerichtete Flüssigkeitsstrahl erzeugt eine
Abwärtsströmung im Leitrohr, die nach Verlassen des Leitrohrs im unteren Reaktorbereich umgelenkt wird und im Ringraum zwischen Leitrohr und Reaktorinnenwand nach oben gerichtet ist. Am oberen Ende des Leitrohrs wird die Flüssigkeit durch den Antriebsstrahl erneut angesaugt, mit diesem vermischt und wieder nach unten gelenkt. Die Schlaufenströmung kann durch die Zugabe von einem oder mehreren gasförmigen Reaktanden in den Ringraum zwischen Leitrohr und Reaktorinnenwand durch Ausnutzung der Gasauftriebskraft unterstützt werden.

Durch den Flüssigkeitsstrahl wird das in der Zweiphasenströmung in Form von Gasblasen vorhandene zirkulierende Gas am oberen Ende des Leitrohrs, in der Nähe der Strahldüse, dispergiert, wodurch sehr hohe Stoffübergangskoeffizienten und volumenspezifische Phasengenzflächen erreicht werden. Weiterhin wird durch den Flüssigkeitsstrahl das Gas aus dem Gasabscheideraum im oberen Reaktorteil angesaugt und dispergiert.

Die Vorrichtung und das Verfahren gemäß der Erfindung sind für insbesondere stark exotherme Gas-Flüssig-, Flüssig-Flüssig- oder Gas-Flüssig-Fest-Reaktionen, vorzugsweise für suspensionskatalysierte Hydrierungen, organische Oxidierungen, Ethoxilierungen, Propoxilierungen oder Aldolkondersationen geeignet.

Die Anwendung des Verfahrens und der Vorrichtung gemäß der Erfindung auf die obengenannten Reaktionen ermöglicht bessere Raumzeitausbeuten, mildere Reaktionsbedingungen, insbesondere bezüglich des Druckes sowie gegebenenfalls der Temperatur, verminderte Reaktionsvolumen, niedrigere Fertigungskosten durch Energiesparung, gegebenenfalls niedrigere Investitionskosten sowie verbesserte Sicherheitsaspekte.

Die Erfindung wird im folgenden anhand einer Figur und eines Ausführungsbeispiels näher erläutert.

Die einzige Figur zeigt einen Längschnitt durch einen erfindungsgemäßen Reaktor 1 mit einer Strahldüse 2 im oberen Reaktorbereich, über die die Edukte und das Reaktionsgemisch zugeführt werden sowie mit in einem Abzug 3 im unteren Reaktorbereich, über den das Reaktionsgemisch in einem äußeren Kreislauf mittels einer Pumpe P der Strahldüse 2 erneut zugeführt wird. Im Reaktor 1 ist ein konzentrisches Leitrohr 4 angeordnet, das sich über den größten Teil der Reaktorlänge, mit Ausnahme der Reaktorenden, erstreckt. Die Figur zeigt unterhalb des unteren Endes des Leitrohres 4 eine Prallplatte 7, die bevorzugt vorgesehen sein kann. Ebenso ist ein Wärmetauscher vorgesehen, der in der Figur als sogenannter Fieldrohr-Wärmetauscher mit Zuführraum 5 und Abführraum 5' für das Kühlmittel K sowie mit Wärmetauscherrohren 6 dargestellt ist. Gemäß einer weiteren Ausführungsform kann der Wärmetauscher in Form von zwischen Böden eingeschweißten Wärmetauscherrohren, die parallel zum Leitrohr 4 angeordnet sind, ausgebildet sein.

### Ausführungsbeispiel

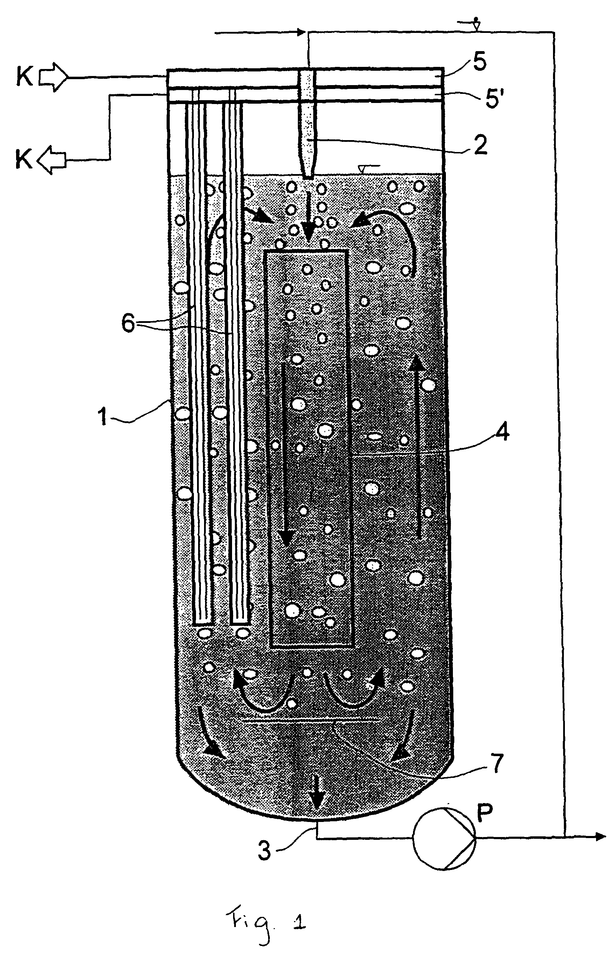

In einem Reaktor gemäß Figur 1 mit einem Reaktionsvolumen von etwa 0,05 m³, der mit 36 parallel angeordneten Fieldrohren versehen ist, die insgesamt eine Kühlfläche von etwa 2,5 m³ aufweisen und in die ein Kühlwasserstrom von etwa 1m³/h und einer Temperatur von etwa 35°C eingeleitet wird, werden mittels einer Dosierpumpe 30,3 kg/h Propionaldehyd bei 75°C in ein schnell fließendes Gemisch aus etwa 84 % Propionsäure, circa 15 % Propionaldehyd und maximal 1 % Nebenprodukte, insbesondere organische Säuren und Alkohole, eingebracht. Durch gleichzeitiges Einleiten von 31 Nm3/h Luft wird im Reaktor ein Druck von 23 bar eingestellt. Zur Aufrechterhaltung der Schlaufenströmung wird im externen Produktkreislauf ein Volumenstrom von 2,5 m3/h umgewälzt. In der Strahldüse herrscht ein Druck von rund 3 bar über Reaktordruck, der spezifische Leistungseintrag beträgt etwa 5 kW/m³.

Die Reaktion verläuft unter nahezu isothermen Bedingungen, da die Reaktionswärme am Ort ihrer Entstehung abgerührt wird. Die maximale Reaktionstemperatur im unteren Drittel des Reaktors beträgt 76°C.

Ein Teilstrom von 38,8 kg/h des Reaktionsgemisches mit 84 % Propionsäure, circa 15 % Propionaldehyd und weniger al 1 % Nebenprodukten, insbesondere organische Säuren und Alkoholen, wird kontinuierlich abgezogen. Dies entspricht einer Raumzeitausbeute von 650 kg/m³h. Der nicht umgesetzte Propionaldehyd kann thermisch, insbesondere destillativ abgetrennt oder in einer Kaskade von zwei hintereinander geschalteten erfindungsgemäßen Reaktoren unter zusätzlicher Verwendung eines Reaktionsrohres weiter abreagiert werden. Der Gesamtumsatz an Propionaldehyd beträgt bei Verwendung einer Kaskade von zwei erfindungsgemäßen Reaktoren 98,7 % bei einer Selektivität von ca. 96 %.

## Patentansprüche

1. Reaktor (1) in hochzylindrischer Bauform zur kontinuierlichen Durchführung von Gas-Flüssig-, Flüssig-Flüssig- oder Gas-Flüssig-Fest-Reaktionen, mit einer im oberen Reaktorbereich angeordneten, nach unten gerichteten Strahldüse (2), über die die Edukte und das Reaktionsgemisch zugeführt werden sowie mit einem Abzug (3) bevorzugt im unteren Reaktorbereich, über den das Reaktionsgemisch in einem äußeren Kreislauf mittels einer Pumpe (P) der Strahldüse (2) erneut zugeführt wird, wobei im Reaktor (1) ein konzentrisches Leitrohr (4) angeordnet ist, das sich im wesentlichen über die gesamte Länge des Reaktors (1) mit Ausnahme der Reaktorenden erstreckt und eine Querschnittsfläche im Bereich von einem Zehntel bis zur Hälfte der Querschnittsfläche des Reaktors (1) aufweist und wobei die Strahldüse (2) oberhalb des oberen Endes des Leitrohrs (4), bevorzugt um ein Achtel des Leitrohrdurchmessers bis zu einem Leitrohrdurchmessers beabstandet, angeordnet ist oder in das Leitrohr (4), in eine Tiefe bis zu mehreren Leitrohrdurchmessern, eintaucht, und wobei in den Ringraum ein Wärmetauscher mit Wärmetauscherrohren integriert ist, **dadurch gekennzeichnet, daß** die Wärmetauscherrohre parallel zum Leitrohr (4) angeordnet sind.

2. Reaktor (1) nach Anspruch 1, **dadurch gekennzeichnet, dass** die Wärmetauscherrohre des Wärmertauschers zwischen Böden eingeschweißt sind.

3. Reaktor (1) nach Anspruch 1 oder 2, **dadurch gekennzeichnet, daß** die Strahldüse (2) als Einstoffdüse ausgebildet ist und daß gegebenenfalls zusätzlich eine Vorrichtung für die Zuführung von einem oder mehreren gasförmigen Reaktanden, bevorzugt ein oder mehrere, insbesondere ein bis drei Ringrohre, mit einer Vielzahl von Öffnungen, insbesondere im unteren Reaktorbereich oder über die Reaktorhöhe verteilt, in den Ringraum zwischen Leitrohr (4) und Reaktorinnenwand, vorgesehen ist (sind).

4. Reaktor (1) nach Anspruch 1 oder 2, **dadurch gekennzeichnet, daß** die Strahldüse (2) als Dreistoff- oder Zweistoffdüse ausgebildet ist.

5. Reaktor (1) nach einem der Ansprüche 1 bis 4, **gekennzeichnet durch** einen Schlankheitsgrad l/d von drei bis zehn, bevorzugt von sechs bis zehn.

6. Reaktor (1) nach einem der Ansprüche 1 oder 5, **dadurch gekennzeichnet, daß** das Verhältnis der Durchmesser von Leitrohr (4) und Reaktor (1) im Bereich von 0,25 bis 0,5, besonders bevorzugt im Bereich von 0,28 bis 0,33, liegt.

7. Reaktor (1) nach einem Ansprüche von 1 bis 6, **dadurch gekennzeichnet, daß** im Reaktorbereich unterhalb des unteren Endes des Leitrohrs (4) eine Prallplatte (7), bevorzugt um einen Leitrohrdurchmesser beabstandet, angeordnet ist.

8. Kontinuierliches Verfahren zur Durchführung von Gas-Flüssig-, Flüssig-Flüssig- oder Gas-Flüssig-Fest-Reaktionen in einem Reaktor (1) nach einem der Ansprüche 1 bis 7, **dadurch gekennzeichnet, daß** der überwiegende Teil des Reaktionsgemisches, entsprechend dem 2- bis 30-fachen, bevorzugt dem 5- bis 10-fachen des Volumenstroms des extern umgepumpten Reaktionsgemisches in einer internen Schlaufenströmung das Leitrohr (4) von oben nach unten und den Ringraum zwischen Leitrohr (4) und Reaktorinnenwand von unten nach oben durchströmt.

9. Verwendung der Vorrichtung nach einem der Ansprüche 1 bis 7 oder des Verfahrens nach Anspruch 8 für stark exotherme Gas-Flüssig-, Flüssig-Flüssig- oder Gas-Flüssig-Fest-Reaktionen, vorzugsweise für Hydrierungen, Oxidierungen, Ethoxilierungen, Propoxilierungen, Hydroformylierungen oder Aldolkondensationen.

## Claims

1. A reactor (1) of high cylindrical construction for continuously carrying out gas-liquid-solid reactions, provided with a downward-directed jet nozzle (2) via which the starting materials and the reaction mixture are fed in and which is located in the upper region of the reactor, and provided with an offtake (3), preferably in the lower region of the reactor, via which the reaction mixture is conveyed via an external circuit back to the jet nozzle (2) by means of a pump (P), where a guide tube (4) which extends essentially over the total length of the reactor (1) with the exception of the reactor ends and has a cross-sectional area in the range from one tenth to one half of the cross-sectional area of the reactor (1) is located concentrically in the reactor (1), and the jet nozzle (2) is located above the upper end of the guide tube (4), preferably at a distance of from one eighth of the guide tube diameter to one guide tube diameter, or projects into the guide tube (4) to a depth up to a plurality of guide tube diameters, and a heat exchanger provided with heat exchange tubes is integrated into the annular space, wherein the heat exchanger tubes run parallel to the guide tube (4).

2. A reactor (1) as claimed in claim 1, wherein the heat exchanger tubes of the heat exchanger are welded in between plates.

3. A reactor (1) as claimed in claim 1 or 2, wherein the jet nozzle (2) is configured as a single-fluid nozzle and, optionally, a facility for introducing one or more gaseous reactants, preferably one or more, in particular from one to three, annular pipes having a plurality of orifices, in particular in the lower region of the reactor or distributed over the height of the reactor, is (are) additionally provided in the annular space between guide tube (4) and interior wall of the reactor.

4. A reactor (1) as claimed in claim 1 or 2, wherein the jet nozzle (2) is configured as a three-fluid or two-fluid nozzle.

5. A reactor (1) as claimed in any of claims 1 to 4, which has an aspect ratio l/d of from 3 to 10, preferably from 6 to 10.

6. A reactor (1) as claimed in any claims 1 to 5, wherein the ratio of the diameters of guide tube (4) and reactor (1) is in the range from 0.25 to 0.5, particularly preferably in the range from 0.28 to 0.33.

7. A reactor (1) as claimed in any of claims 1 to 6, wherein an impingement plate (7) is located in the region of the reactor below the lower end of the guide tube (4), preferably at a distance of one guide tube diameter.

8. A continuous process for carrying out gas-liquid or gas-liquid-solid reactions in a reactor (1) as claimed in any of claims 1 to 7, wherein the major part of the reaction mixture, corresponding to from 2 to 30 times, preferably from 5 to 10 times, the volume flow of the reaction mixture pumped around the external circuit flows in an internal loop through the guide tube (4) from the top downward and through the annular space between guide tube (4) and interior wall of the reactor from the bottom upward.

9. The use of an apparatus as claimed in any of claims 1 to 7 or a process as claimed in claim 8 for strongly exothermic gas-liquid or gas-liquid-solid reactions, preferably for hydrogenations, oxidations, ethoxylations, propoxylations, hydroformylations or aldol condensations.

## Revendications

1. Réacteur (1), ayant la forme d'un cylindre de grande hauteur, pour la mise en oeuvre continue de réactions gaz-liquide, liquide-liquide ou gaz-liquide-solide, comportant une buse d'injection (2), disposée dans la zone supérieure du réacteur et dirigée vers le bas, buse d'injection qui assure l'amenée des matières de charge et du mélange réactionnel, ainsi qu'une décharge (3), de préférence dans la zone inférieure du réacteur, par laquelle le mélange réactionnel est renvoyé à la buse d'injection (2) à l'aide d'une pompe (P) dans un circuit extérieur fermé, un tube directeur concentrique (4) étant disposé dans le réacteur (1), ce tube s'étendant pour l'essentiel sur toute la longueur du réacteur (1) à l'exception des extrémités du réacteur, et présentant une aire en section transversale comprise entre un dixième et la moitié de l'aire en section transversale du réacteur (1), la buse d'injection (2) étant disposée au-dessus de l'extrémité supérieure du tube directeur (4), de préférence à une distance comprise entre un huitième et un diamètre de tube directeur, ou encore étant immergée dans le tube directeur (4), sur une profondeur allant jusqu'à plusieurs diamètres du tube directeur, un échangeur de chaleur comportant des tubes échangeurs de chaleur étant intégré dans l'espace annulaire, **caractérisé en ce que** les tubes échangeurs de chaleur sont parallèles au tube directeur (4).

2. Réacteur (1) selon la revendication 1, **caractérisé en ce que** les tubes échangeurs de chaleur de l'échangeur de chaleur sont soudés entre des fonds.

3. Réacteur (1) selon la revendication 1 ou 2, **caractérisé en ce que** la buse d'injection (2) est configurée comme une buse à un composant, et **en ce que** l'on prévoit éventuellement en outre un dispositif pour amener dans l'espace annulaire situé entre le tube directeur (4) et la paroi intérieure du réacteur un ou plusieurs réactifs gazeux, de préférence un ou plusieurs et en particulier un à trois tubes annulaires comportant un grand nombre d'ouvertures réparties en particulier dans la zone inférieure du réacteur ou sur toute la hauteur du réacteur.

4. Réacteur (1) selon la revendication 1 ou 2, **caractérisé en ce que** la buse d'injection (2) est configurée comme une buse à trois ou deux composants.

5. Réacteur (1) selon l'une des revendications 1 à 4, **caractérisé par** un élancement 1/d de trois à dix, de préférence de six à dix.

6. Réacteur (1) selon l'une des revendications 1 ou 5, **caractérisé en ce que** le rapport du diamètre du tube directeur (4) au réacteur (1) est compris dans la plage de 0,25 à 0,5 et plus particulièrement dans la plage de 0,28 à 0,33.

7. Réacteur (1) selon l'une des revendications 1 à 6, **caractérisé en ce que**, dans la zone du réacteur située en dessous de l'extrémité inférieure du tube directeur (4), est disposée une plaque chicane (7), qui de préférence se trouve à une distance d'un diamètre de tube directeur.

8. Procédé continu pour mettre en oeuvre des réactions gaz-liquide, liquide-liquide ou gaz-liquide-solide dans un réacteur (1) selon l'une des revendications 1 à 7, **caractérisé en ce que** la plus grande partie du mélange réactionnel, correspondant à 2 à 30 fois et de préférence 5 à 10 fois le débit volumique du mélange réactionnel mis en recirculation externe par pompage, passe par un écoulement interne en boucle dans le tube directeur (4), de haut en bas, et dans l'espace annulaire entre le tube directeur (4) et la paroi intérieure du réacteur de bas en haut.

9. Utilisation du dispositif selon l'une des revendications 1 à 7 ou du dispositif selon la revendication 8 pour des réactions gaz-liquide, liquide-liquide ou gaz-liquide-solide fortement exothermiques, de préférence pour des hydrogénations, des oxydations, des éthoxylations, des propoxylations, des hydroformylations ou des condensations des aldols.
